(19) Europäisches Patentamt
European Patent Office
Office européen des brevets

(11) Veröffentlichungsnummer : **0 061 669**
**B1**

(12) # EUROPÄISCHE PATENTSCHRIFT

(45) Veröffentlichungstag der Patentschrift : **25.07.84**

(51) Int. Cl.³ : **C 07 C 45/45**, C 07 C 49/427, C 07 C 49/403, C 07 C 49/657, C 07 C 49/443

(21) Anmeldenummer : **82102269.6**

(22) Anmeldetag : **19.03.82**

(54) **Verbessertes Verfahren zur Herstellung von Cyclohexan-1,3-dionen sowie einige neue bicyclische Cyclohexan-1,3-dione.**

(30) Priorität : **27.03.81 DE 3112056**

(43) Veröffentlichungstag der Anmeldung : **06.10.82 Patentblatt 82/40**

(45) Bekanntmachung des Hinweises auf die Patenterteilung : **25.07.84 Patentblatt 84/30**

(84) Benannte Vertragsstaaten : **CH DE FR GB LI NL**

(56) Entgegenhaltungen :
**DE-A- 2 533 919**
**JOURNAL OF ORGANIC CHEMISTRY, Band 22, 1957, Seiten 1268-1269 J.J. MILLER et al.: "Reaction of ethyl aerylate with methyl n-hexyl ketone"**
**CHEMICAL ABSTRACTS, Band 84, Nr. 19, 10. Mai 1976, Seite 457, Spalte 2, Nr. 135187w, Columbus, Ohio, USA**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Baumann, Manfred, Dr.**
**Im Sennteich 26**
**D-6800 Mannheim (DE)**
Erfinder : **Hoffmann, Werner, Dr.**
**Ringstrasse 11 C**
**D-6701 Neuhofen (DE)**

**EP 0 061 669 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein verbessertes Verfahren zur Herstellung von Cyclohexan-1,3-dionen sowie einige neue bicyclische Cyclohexan-1,3-dione. Durch Dehydrierung lassen sich die Cyclohexan-1,3-dione in Resorcine überführen, die als Kunstharzkomponenten in der Gummi- und Holzleimindustrie, als Kupplungskomponenten für Farbstoffe oder als Antiseptika Verwendung finden.

Es hat daher nicht an Versuchen gefehlt, die Cyclohexan-1,3-dione auf möglichst vorteilhafte Weise herzustellen. So sind verschiedene Methoden zur Herstellung derselben aus Ketonen und Acrylestern bekannt.

Beispielsweise ist es bekannt, Ketone aminkatalysiert an Acrylester zu addieren und die dabei erhaltenen 4-Oxocarbonsäurealkylester in der Flüssigphase (vgl. DE-OS 22 45 270 bzw. DE-OS 25 33 920) oder in der Gasphase (vgl. DE-OS 24 12 313) zu den Cyclohexandionen zu cyclisieren. Nachteilig an diesem Verfahren ist, daß zwei technisch aufwendige Reaktionsstufen erforderlich sind.

Auch 5-Ketonitrile lassen sich in Gegenwart von Schwefelsäure oder Phosphorsäure zu Cyclohexan-1,3-dionen cyclisieren (vgl. DE-OS 21 44 170). Jedoch ist hierbei die Verwendung großer Mengen Schwefelsäure bzw. Phosphorsäure sowie die Notwendigkeit von zwei getrennten aufwendigen Reaktionsschritten technisch nachteilig.

Weiterhin ist es aus J. Org. Chem. 1957, Seite 1268 bekannt 4-Pentyl-cyclohexan-1,3-dion in einer einstufigen Synthese durch Umsetzen von Methylhexylketon und Acrylsäureethylester in Gegenwart von Natriumethylat in Xylol herzustellen. Gemäß diesem Verfahren wird zu einer Suspension von Natriumethoxid in Xylol, welche durch Zugabe von Ethanol zu einer Suspension von Natrium in Xylol hergestellt wurde, Acrylsäureethylester zugefügt, das Reaktionsgemisch in einem Eisbad gekühlt, tropfenweise mit Methylhexylketon versetzt, dann noch 0,5 h bei 0 °C und 17 h bei Raumtemperatur gerührt. Ein wesentlicher Nachteil dieses Verfahrens ist die geringe Selektivität von 27 %, die keine wirtschaftliche Produktion zuläßt.

Es wurde daher vorgeschlagen (vgl. DE-OS 25 33 919), die Cyclohexan-1,3-dione durch Umsetzen von Acrylsäureestern mit Ketonen in flüssiger Phase, in Gegenwart einer starken Base und in Gegenwart ganz bestimmter Lösungsmittel herzustellen. Als Lösungsmittel wurden Carbonsäureamide, Phosphorsäureamide, Sulfoxide, Sulfone sowie zahlreiche Ether mehrwertiger Alkohole genannt. Gemäß diesem Verfahren werden die Cyclohexandione in Ausbeuten von 33 bis 85 %, bezogen auf umgesetzten Acrylester, und von 52 bis 87,5 %, bezogen auf umgesetztes Keton erhalten. Nachteilig an diesem Verfahren ist vor allem, daß die notwendigen Lösungsmittel relativ teuer, teilweise schwierig zu handhaben und nach der Reaktion schwierig zurückzugewinnen sind. Weiterhin ist die Aufarbeitung des Reaktionsgemisches ziemlich aufwendig.

Zum Vergleich wurde in der genannten DE-OS die Umsetzung von Acrylsäureestern mit Ketonen in einem üblichen unpolaren Lösungsmittel durchgeführt. Hierbei wurde bei der entsprechenden Umsetzung von Aceton mit Isopropylacrylat in Xylol gar kein Cyclohexandion erhalten (s. Vergleichsbeispiel 2) und bei der Umsetzung von 2-Octanen mit Ethylacrylat in Xylol das 4-Pentyl-1,3-cyclohexandion in Selektivitäten von etwa 35 % gebildet.

Es war die Aufgabe der Erfindung, das Verfahren zur Herstellung von Cyclohexan-1,3-dionen durch Umsetzen von Acrylestern mit Ketonen so zu verbessern, daß man trotz Verwendung von billigeren und leichter rückgewinnbaren Lösungsmitteln gleich gute, oder bessere Ausbeuten erzielen kann und die Isolierung und Reinigung der Reaktionsprodukte erleichtert wird.

Gegenstand der Erfindung ist ein verbessertes Verfahren zur Herstellung von Cyclohexan-1,3-dionen der allgemeinen Formel I

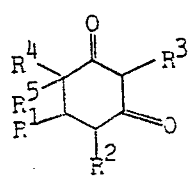

(I)

in der die Reste $R^1$, $R^3$ und $R^4$ Wasserstoff, Alkylgruppen mit 1 bis 10 C-Atomen, vorzugsweise eine Methyl- oder Ethylgruppe oder eine Arylgruppe, vorzugsweise eine Phenylgruppe bedeuten, der Rest $R^2$ für Wasserstoff oder eine Alkylgruppe mit 1 bis 10 C-Atomen, vorzugsweise eine Methylgruppe steht, der Rest $R^5$ für eine Alkylgruppe mit 1 bis 10 C-Atomen, vorzugsweise eine Methyl- oder Ethylgruppe oder eine Arylgruppe, vorzugsweise eine Phenylgruppe steht, oder $R^4$ und $R^5$ zusammen für einen Alkylenrest mit 2 bis 10 C-Atomen stehen, oder aber $R^4$ oder $R^5$ und $R^3$ zusammen für einen Alkylenrest mit 2 bis 12 C-Atomen stehen, durch Umsetzen von Acrylsäureestern der allgemeinen Formel II

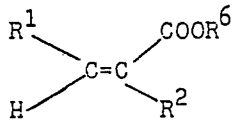

(II)

in der $R^6$ für eine Alkylgruppe mit 1 bis 4 C-Atomen, vorzugsweise eine Methylgruppe steht und $R^1$ und $R^2$ die oben angegebene Bedeutung haben, mit Ketonen der allgemeinen Formel III

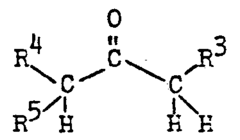

(III)

in der $R^3$ bis $R^5$ die oben angegebene Bedeutung haben, in der Flüssigphase in Gegenwart eines stark basischen Kondensationsmittels sowie in Gegenwart eines üblichen unpolaren Lösungsmittels, das dadurch gekennzeichnet ist, daß das Reaktionsgemisch zusätzlich noch etwa 0,01 bis 5, vorzugsweise 0,1 bis 4 Mol eines Alkanols mit 1 bis 4 C-Atomen pro Mol des jeweils im Reaktionsgemisch vorhandenen Acrylsäureesters enthält und daß man das Reaktionsgemisch während und/oder nach der Zugabe der Reaktionspartner auf Temperaturen von 40 bis 200 °C, vorzugsweise 90 bis 140 °C erwärmt und/oder wenn man den Acrylsäureester in Form seines Additionsproduktes mit einem Alkanol mit 1 bis 4 C-Atomen der allgemeinen Formel IV

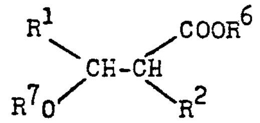

(IV)

in dem $R^7$ Alkyl mit 1 bis 4 C-Atomen, vorzugsweise Methyl oder Ethyl bedeutet, verwendet und das Reaktionsgemisch auf Temperaturen von 40 bis 200 °C, vorzugsweise 90 bis 140 °C erwärmt.

Die Additionsprodukte der Acrylsäureester mit einem Alkanol bilden sich sofort und quanitativ bei der Umsetzung der Acrylsäureester mit dem Alkanol in Gegenwart katalytischer Mengen einer Base und dementsprechend auch unter den Reaktionsbedingungen der erstgenannten Variante des erfindungsgemäßen Verfahrens.

Besonders vorteilhaft verläuft das erfindungsgemäße Verfahren, wenn man bei der Umsetzung von Acrylsäureestern der Formel II mit den Ketonen der Formel III das Reaktionsgemisch nach der Zugabe der Reaktionspartner unter Abdestillieren des im Reaktionsgemisch vorhandenen Alkanols erhitzt, bzw. wenn man bei der Umsetzung von Acrylsäureester-Additionsprodukten der Formel IV mit Ketonen der Formel III das Reaktionsgemisch unter Abdestillieren des im Reaktionsgemisch vorhandenen Alkanols erhitzt.

Es war überraschend, daß man die Cyclohexan-1,3-dione auf die einfache erfindungsgemäße Weise in sehr guten Ausbeuten erhalten kann, während die entsprechende Umsetzung sowohl in einem unpolaren Lösungsmittel als auch in einem Alkanol als alleinigem Lösungsmittel nur mit äußerst unbefriedigenden Ausbeuten verläuft (s. Vergleichsbeispiel).

Gegenstand der Erfindung sind außerdem die neuen bicyclischen Cyclohexan-1,3-dione : Bicyclo [9.3.1] pentadecan-14,15-dion und 13-Methyl-bicyclo [9.3.1] pentadecan-14,15-dion und 12-Methyl-bicyclo [9.3.1] pentadecan-14,15-dion.

Die als Ausgangsverbindungen für das erfindungsgemäße Verfahren benötigten Acrylsäureester und Ketone sind bekannte Verbindungen, die teils handelsüblich sind und teils auf bekannte Weise erhalten werden können.

Die als Alkylgruppen definierten Substituenten in den als Ausgangsverbindungen benutzten Acrylestern und Ketonen können geradkettig, verzweigt oder auch cyclisch sein.

Die als Arylgruppen definierten Substituenten haben im allgemeinen bis zu 14 C-Atome und können auch durch niedere Alkylgruppen substituiert sein. Bevorzugt sind der Phenyl und der Naphthylrest, insbesondere der Phenylrest.

Als Ausgangsprodukte besonders geeignete Ketone sind Aceton, Methylethylketon, Methylpropylketon, Methylisopropylketon, Methylisobutylketon, Diethylketon, Methylbutylketon, Methylpentylketon, Methylhexylketon, Cyclopentanon, Cyclohexanon, 2-Methylcyclohexanon, 4-Methylcyclohexanon, n-Propyl-isopropylketon, 1,3-Diphenylaceton, 1,1-Diphenyl-aceton, Cyclododecanon und Ethylisopropylketon.

Als α,β-ungesättigte Carbonsäureester sind besonders geeignet der Methyl-, Ethyl-, Butyl-, Isopropyl- und Isobutylester von Acrylsäure, Methacrylsäure, Crotonsäure und Zimtsäure.

Die Ausgangsverbindungen der Formeln II und III verwendet man mit Vorteil in etwa äquimolekularen Mengen. Ein Überschuß an Keton ist möglich, jedoch im allgemeinen nicht nötig ; ein größerer Überschuß an Acrylester ist nicht empfehlenswert, da überschüssiger Acrylester polymerisieren kann.

Als starke Basen kann man die Alkali- und Erdalkali-Alkoholate, die -Oxide, sowie die Metalle selbst verwenden.

Vorzugsweise werden die Alkali- und Erdalkali-Alkoholate, insbesondere Natriummethylat verwendet, das technisch als Festsubstanz oder als 30 gew.%ige methanolische Lösung billig verfügbar ist.

Die Base verwendet man im allgemeinen in molaren Mengen bis zu einem etwa 10 %igen molaren Überschuß.

Als übliche unpolare Lösungsmittel verstehen wir im Rahmen der vorliegenden Erfindung insbesondere Kohlenwasserstoffe oder chlorierte Kohlenwasserstoffe mit einem Siedepunkt zwischen etwa 40 und 200 °C, wie beispielsweise Xylol, Toluol, Methylenchlorid, Chlorbenzol und Dichlorbenzol sowie einige praktisch unpolare Ether, wie z. B. Tetrahydrofuran und Methyl-tert.-butylether. Bevorzugt werden die Lösungsmittel mit einem Siedepunkt zwischen 90 und 160 °C, wie Xylol, Toluol und Chlorbenzol.

Die erfindungsgemäß verwendeten Lösungsmittel sind im allgemeinen billiger, leichter zu handhaben und wesentlich leichter zurückgewinnbar als die gemäß den bekannten Verfahren verwendeten Lösungsmittel.

Als Alkanole, die dem Lösungsmittel zugesetzt werden, verwendet man im allgemeinen Alkanole mit 1 bis 4 C-Atomen, vorzugsweise Methanol oder Ethanol, insbesondere Methanol, das technisch besonders billig verfügbar ist.

Die Alkanole verwendet man im allgemeinen in Mengen von 0,01 bis 5, vorzugsweise 0,1 bis 4 Mol pro Mol des jeweils im Reaktionsgemisch vorhandenen Acrylsäureesters.

Mit besonderem Vorteil führt man das erfindungsgemäße Verfahren so aus, daß man zu einer Mischung aus dem basischen Kondensationsmittel, dem Lösungsmittel und dem Alkanol gleichzeitig etwa gleiche molare Mengen an dem Acrylsäureester II und dem Keton III einträgt und das Reaktionsgemisch während und/oder nach der Zugabe auf die beanspruchten Temperaturen erhitzt. Auf diese Weise kommt man mit minimalen Methanolmengen zu sehr guten Erfolgen. Verwendet man größere Mengen Alkanol, empfiehlt es sich, dieses sowie das bei der Umsetzung sich bildende Alkanol im Laufe der Umsetzung abzudestillieren, denn die Umsetzung verläuft in Methanol als Lösungsmittel nur sehr schlecht (s. Vergleichsbeispiel 3).

Ebenso vorteilhaft verläuft das erfindungsgemäße Verfahren, wenn man zu einer Mischung aus dem basischen Kondensationsmittel und dem Lösungsmittel gleichzeitig etwa gleiche molare Mengen an dem Acrylsäureester-Alkanoladditionsprodukt der Formel IV und dem Keton III einträgt und das Reaktionsgemisch während und/oder nach der Zugabe auf die beanspruchten Temperaturen erhitzt.

Die Äquivalenz beider Verfahrensvarianten erscheint plausibel, wenn man bedenkt, daß aus Acrylsäureestern und Alkanolen in Gegenwart von Basen, wie sie unter den Reaktionsbedingungen der Erfindung vorliegen, Acrylsäureester-Alkanoladditionsprodukte gebildet werden.

Eine mögliche Erklärung für den unerwartet vorteilhaften Verlauf des erfindungsgemäßen Verfahrens liegt in Folgendem. Vermutlich wird unter den Reaktionsbedingungen zunächst das Acrylester-Alkanoladditionsprodukt gebildet. Aus diesem wird dann basenkatalysiert freier Acrylester in geringer Konzentration zurückgebildet, der dann mit dem in relativ höherer Konzentration vorliegenden Keton abreagieren kann, ohne daß Eigenkondensationsreaktionen und/oder Polymerisationsreaktionen stattfinden können, die üblicherweise die Ausbeute an Cyclohexandionen vermindern.

Auch die Aufarbeitung des Reaktionsgemisches gestaltet sich bei dem erfindungsgemäßen Verfahren besonders einfach. Gewünschtenfalls kann das bei der Umsetzung zunächst aus dem Reaktionsgemisch ausfallende Alkalisalz des Enols von dem erhaltenen Cyclohexandion einfach durch Abfiltrieren isoliert und in dieser Form weiterverwendet werden.

Zur Isolierung der Cyclohexandione selbst wird das erhaltene Reaktionsgemisch mit Wasser vermischt. Die unumgesetzten Ausgangsverbindungen sowie nicht saure Nebenprodukte oder Verunreinigungen befinden sich dann in der organischen Phase, die leicht abgetrennt und durch Destillation aufgearbeitet werden kann. Die gewünschten Reaktionsprodukte befinden sich in Form von Salzen in der wäßrigen Phase und können durch Ansäuern daraus gewonnen werden. Die erhaltenen Produkte sind im allgemeinen so sauber, daß keine weitere Reinigung erforderlich ist, sie enthalten teilweise jedoch noch geringe Mengen Wasser.

Gemäß dem Verfahren der Erfindung können die Cyclohexan-1,3-dione der Formel I auf sehr vorteilhafte Weise hergestellt werden. Die Cyclohexan-1,3-dione sind wertvolle Zwischenprodukte, beispielsweise für die Herstellung von Kunstharzkomponenten in der Gummi- und Holzleimindustrie, als Kupplungskomponenten für Farbstoffe oder als Zwischenprodukte für Carotenoide.

Die 3 neuen bicyclischen Cyclohexan-1,3-dione Bicyclo [9.3.1] pentadecan-14,15-dion und 12- bzw. 13-Methylbicyclo [9.3.1] pentadecan-14,15-dion sind wertvolle Zwischenprodukte für die Herstellung von Riechstoffen.

Beispiel 1

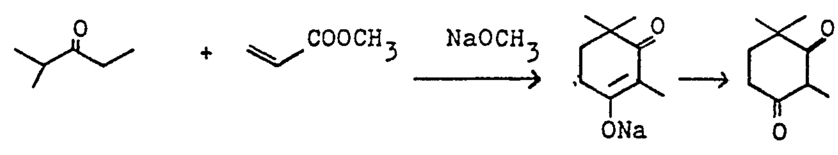


1 800 ml Xylol werden mit 600 g einer 30 %igen methanolischen $NaOCH_3$-Lösung (entsprechend 13,1 Mol Methanol und 3,3 Mol $NaOCH_3$) versetzt. Zu dieser Lösung wird unter gutem Rühren und unter Stickstoff-Schutzgas im Verlauf von 1 Stunde ein Gemisch aus 300 g (3 Mol) Ethylisopropylketon und 258 g (3 Mol) Acrylsäuremethylester zugetropft.

Die Temperatur des Reaktionsgemisches steigt dabei auf ca. 40 °C an. Anschließend wird das Reaktionsgemisch erhitzt und bis zu einer Innentemperatur von 130 °C Methanol abdestilliert. Man erhält ca. 250 g Destillat. Im Reaktionsgefäß liegt dann eine Suspension des Na-Salzes in Xylol vor.

Dieses kann entweder abfiltriert und als solches weiter umgesetzt werden oder folgendermaßen aufgearbeitet werden.

Bei Zugabe von 800 ml $H_2O$ löst sich das Salz auf. Die organische Phase wird abgetrennt. Die wäßrige Phase wird mit halbkonzentrierter Salzsäure bis zu einem pH Wert von 2-3 angesäuert. Das hierbei ausfallende Produkt wird mit Essigester extrahiert, die Lösung getrocknet und eingeengt. Es hinterbleibt ein Rückstand von 430 g. Das zähe Öl erstarrt zu einer wachsartigen Masse.

IR, NMR und Massenspektrum bestätigen, daß es sich um 2,4,4-Trimethyl-cyclohexan-1,3-dion im Gleichgewicht mit seinem Enol handelt. Die Analyse zeigt, daß noch 1/4 Mol Kristallwasser enthalten ist. Ausbeute 92 % der Theorie.

Beispiel 2

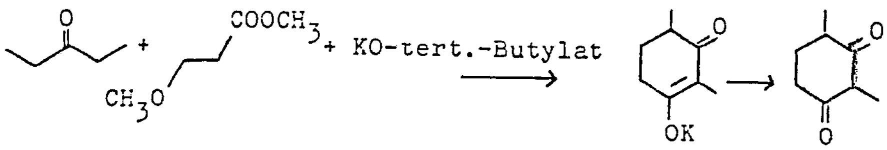


11,5 g Kalium-tert.-Butylat (0,1 Mol) werden unter $N_2$ in 50 ml Xylol suspendiert. Zu dieser Suspension wird in 45 Min. ein Gemisch aus 8,6 g (0,1 Mol) Diethylketon und 11,8 g (0,1 Mol) β-Methoxy-propionsäuremethylester getropft. Die Temperatur des Reaktionsgemisches steigt dabei auf 40 °C an. Anschließend wird noch 2 Stunden auf 90 °C erwärmt.

Nach dem Abkühlen wird das Reaktionsgemisch mit 70 ml $H_2O$ versetzt und wie in Beispiel 1 aufgearbeitet. Man erhält 9 g eines kristallinen Produkts vom Schmp. 105-111 °C.

Gemäß IR-, NMR- und Massenspektren handelt es sich um 2,4-Dimethyl-cyclohexan-1,3-dion im Gleichgewicht mit seinem Enol. Ausbeute 64 %.

Beispiel 3

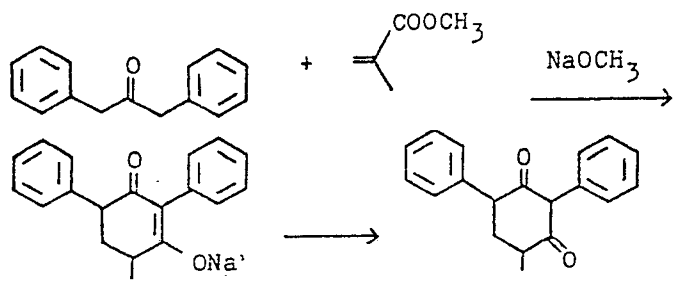


Zu einer Mischung aus 20 g einer 30 %igen $NaOCH_3$-Lösung in Methanol (entsprechend 0,11 Mol $NaOCH_3$ und 0,44 Mol $CH_3OH$) und 50 ml Xylol wird unter $N_2$ die Lösung von 21 g (0,1 Mol) 1,3-Diphenylaceton und 10 g (0,1 Mol) Methacrylsäuremethylester in 70 ml Xylol im Verlauf von 30 Min. zugetropft. Anschließend wird das Reaktionsgemisch analog Beispiel 1 unter Abdestillieren von Methanol erhitzt und wie in Beispiel 1 näher erläutert aufgearbeitet.

Man erhält 26,5 g eines zähen Öls, das beim Anreiben auskristallisiert. F = 115-118 °C. Ausbeute 95,3 % der Theorie.

Gemäß IR-, NMR- und Massenspektrum handelt es sich um 2,4-Diphenyl-6-methyl-cyclohexan-1,3-dion im Gleichgewicht mit seinem Enol.

Beispiel 4

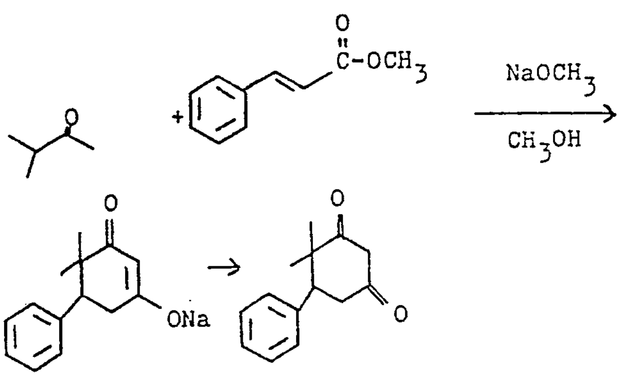

Zu einer Mischung von 20 g einer 30 %igen $NaOCH_3$-Lösung in Methanol (entsprechend 0,11 Mol $NaOCH_3$ und 0,44 Mol $CH_3OH$) in 50 ml Xylol werden unter $N_2$ innerhalb von 30 Min. zunächst 16,2 g (0,1 Mol) Zimtsäuremethylester getropft und anschließend in 40 Min. 9 g (0,1 Mol) Isopropylmethylketon. Anschließend wird das Reaktionsgemisch zum Sieden erhitzt und bis zu einer Innentemperatur von 120 °C Methanol abdestilliert.

Die Aufarbeitung erfolgt wie in Beispiel 1 näher beschrieben. Man erhält 17,1 g einer Festsubstanz vom Schmelzpunkt F = 182-183 °C. Ausbeute 89 % der Theorie.

Gemäß IR-, NMR- und Massenspektum handelt es sich um 4,4-Dimethyl-5-phenyl-cyclohexan-1,3-dion im Gleichgewicht mit seinem Enol.

Beispiel 5

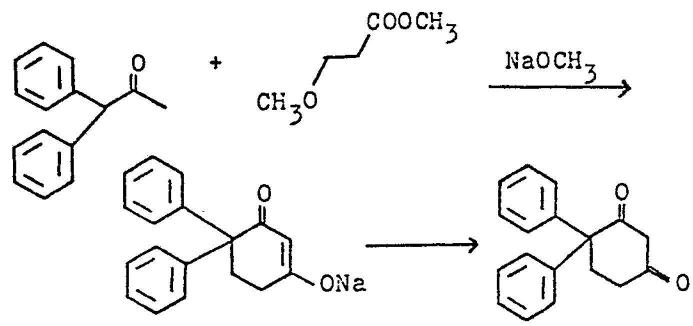


Zu einer Suspension von 5,5 g (0,1 Mol) festem $NaOCH_3$ in 50 ml Toluol wird unter $N_2$-Atmosphäre eine Lösung von 21 g 1,1'-Diphenylaceton und 11,8 g β-Methoxypropionsäuremethylester in 100 ml Toluol innerhalb von 2 Stunden bei 90 °C zugetropft. Es fällt ein voluminöser Niederschlag aus. Das Reaktionsgemisch wird mit 100 ml Wasser versetzt und die erhaltene wäßrige Phase mit 50 %iger $H_2SO_4$ auf einen pH-Wert von 2-3 angesäuert. Der erhaltene Niederschlag wird abfiltriert und getrocknet. Man erhält so 21 g einer Substanz mit einem Schmelzpunkt von 185-187 °C. Ausbeute 79 %.

Gemäß IR-, NMR- und Massenspektrum handelt es sich um 4,4-Diphenyl-cyclohexan-1,3-dion im Gleichgewicht mit seinem Enol.

Beispiel 6

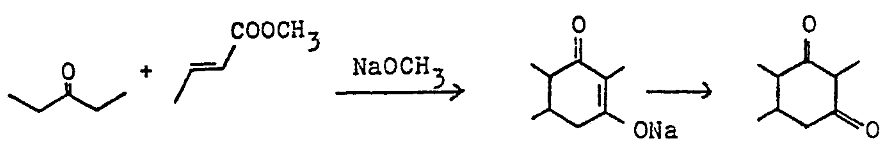


Zu einer Mischung aus 20 g einer 30 %igen $NaOCH_3$-Lösung in Methanol (entsprechend 0,11 Mol $NaOCH_3$ und 0,44 Mol $CH_3OH$) und 70 ml Xylol wird unter gutem Rühren und unter $N_2$-Schutzgasatmosphäre ein Gemisch aus 8,6 g (0,1 Mol) Diethylketon und 10 g (0,1 Mol) Crotonsäuremethylester zugetropft und das Reaktionsgemisch unter Abdestillieren von Methanol erhitzt bis die Innentemperatur des Reaktionsgefäßes 130 °C beträgt. Anschließend wird das Reaktionsgemisch wie in Beispiel 1 näher beschrieben aufgearbeitet. Man erhält das 2,4,5-Trimethyl-cyclohexan-1,3-dion vom Schmelzpunkt F = 121-125 °C in einer Ausbeute von 70 % der Theorie.

Beispiel 7

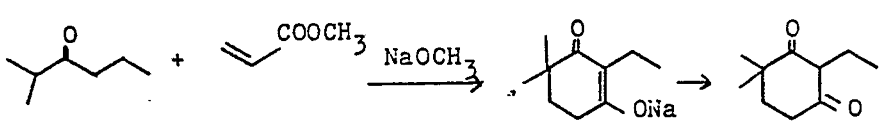


Zu einer Mischung von 20 g einer 30 %igen Lösung von $NaOCH_3$ in Methanol (entsprechend 0,11 Mol $NaOCH_3$ und 0,44 Mol $CH_3OH$) und 70 ml Xylol wird unter Rühren und unter $N_2$-Schutzgas ein Gemisch aus 11,4 g (0,1 Mol) n-Propyl-isopropylketon und 8,6 g (0,1 Mol) Acrylsäuremethylester zugetropft und anschließend das Reaktionsgemisch unter Abdestillieren von Methanol erhitzt, bis die Innentemperatur des Reaktionsgefäßes 130 °C beträgt.

Bei einer Aufarbeitung analog Beispiel 1 erhält man 13 g 2-Ethyl-4,4-dimethyl-cyclohexan-1,3-dion in einer Ausbeute von 77 % der Theorie.

Beispiel 8

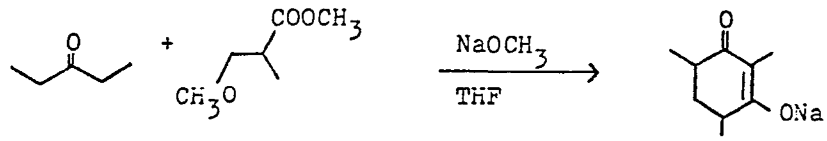


5,5 g (0,1 Mol) $NaOCH_3$ werden in 50 ml THF unter $N_2$ suspendiert. Zu dieser Suspension werden unter Erhitzen bis zum Rückfluß innerhalb von 2 Stunden ein Gemisch aus 8,6 g (0,1 Mol) Diethylketon und 13,2 g (0,1 Mol) 2-Methyl-3-methoxy-propionsäuremethylester zugetropft und das Reaktionsgemisch noch 4,5 Stunden unter Rückfluß zum Sieden erhitzt. Anschließend wird mit Wasser zersetzt, die wäßrige Phase bis zu einem pH-Wert von 2-3 angesäuert und mit Essigester extrahiert und der Extrakt destilliert. Es werden 13 g 2,4,6-Trimethyl-cyclohexen-1,3-dion erhalten. Schmelzpunkt F = 115-120 °C ; Ausbeute = 84 % der Theorie.

Beispiel 9

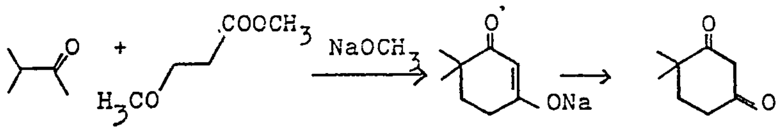


8,6 g (0,1 Mol) Isopropylmethylketon werden mit 11,8 g (0,1 Mol) β-Methoxy-propionsäuremethylester und 0,1 Mol $NaOCH_3$ in Toluol analog Beispiel 5 umgesetzt. Man erhält 11 g 4,4-Dimethyl-cyclohexan-1,3-dion vom Schmelzpunkt F = 87-90 °C. Ausbeute 78 %.

Beispiel 10

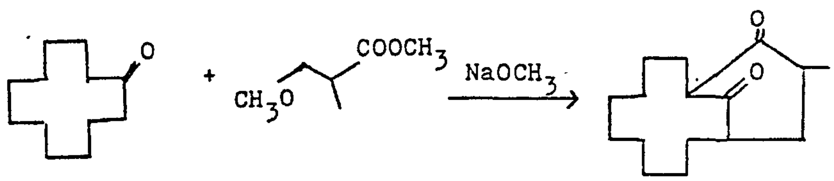


27 g (0,5 Mol) festes $NaOCH_3$ werden unter $N_2$ in 500 ml Toluol suspendiert. Zu dieser Suspension werden bei 90 °C eine Lösung von 91 g (0,5 Mol) Cyclododecanon und 66 g (0,5 Mol) 2-Methyl-3-methoxy-propionsäuremethylester in Toluol zugetropft und das Reaktionsgemisch noch 7 Stunden bei 90 °C gehalten.

Eine Aufarbeitung analog Beispiel 1 liefert aus der Toluolphase 10 g Cyclododecanon zurück und aus der wäßrigen Phase 100 g des neuen 13-Methyl-bicyclo [9.3.1] pentadecan-14,15-dion vom Schmelzpunkt F = 150-155 °C. Die Ausbeute beträgt 94,8 %, bezogen auf umgesetztes Cyclododecanon.

Beispiel 11

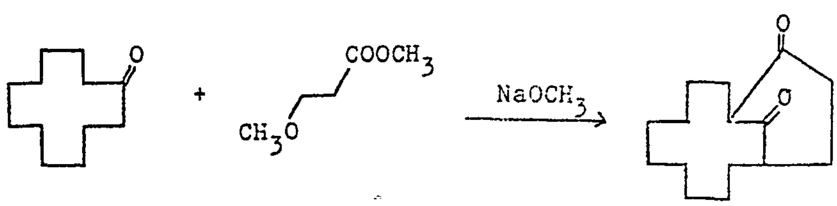


Bei der Umsetzung von 0,5 Mol Cyclododecanon und 0,5 Mol β-Methoxy-propionsäuremethylester in Gegenwart von 0,5 Mol $NaOCH_3$ analog Beispiel 10 erhält man das neue Bicyclo [9.3.1] pentadecan-14,15-dion vom Schmelzpunkt F = 157-159 °C. Ausbeute 60 %, bezogen auf umgesetztes Cyclododecanon.

Beispiel 12

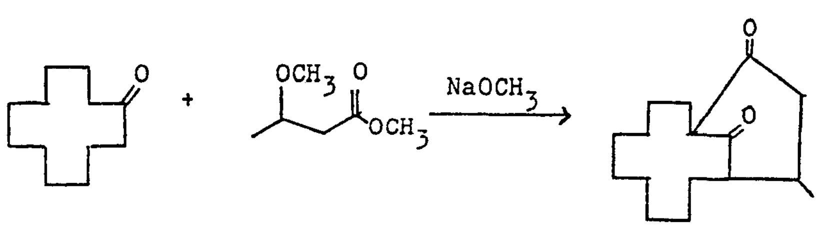

Bei der Umsetzung von 0,5 Mol Cyclododecanon und 0,5 Mol β-Methoxy-buttersäuremethylester in Gegenwart von 0,5 Mol $NaOCH_3$ analog Beispiel 10 wird das neue 12-Methyl-bicyclo [9.3.1]-pentadecan-14,15-dion vom Schmelzpunkt F = 185-191 °C erhalten. Ausbeute 26 %, bezogen auf umgesetztes Cyclododecanon.

## Beispiel 13

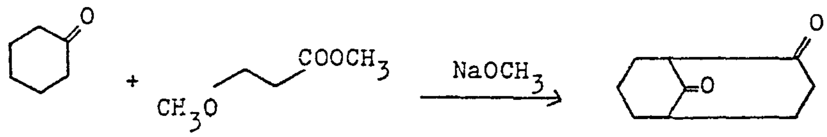


Bei der Umsetzung von 0,1 Mol Cyclohexanon und 0,1 Mol β-Methoxy-propionsäuremethylester in Gegenwart von 0,1 Mol $NaOCH_3$ analog Beispiel 10 wird 13 g Bicyclo [3.3.1] nonan-2,9-dion in Form eines zähen Öls erhalten. Ausbeute 85,5 %.

Das NMR-Spektrum bestätigt die erwartete Struktur.

## Beispiel 14

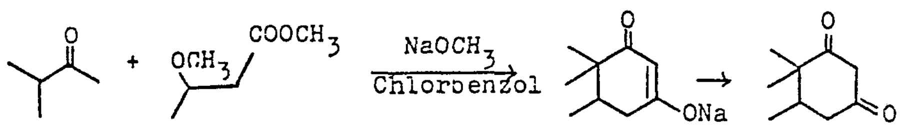


5,5 g (0,1 Mol) $NaOCH_3$ werden in 80 ml Chlorbenzol suspendiert. In diese Suspension wird innerhalb von 1,5 Stunden ein Gemisch aus 8,6 g (0,1 Mol) Methylisopropylketon und 13,2 g (0,1 Mol) β-Methoxy-buttersäuremethylester eingetropft. Anschließend wird das Reaktionsgemisch unter Abdestillieren von Methanol erhitzt bis die Innentemperatur im Reaktionsgemisch 130 °C erreicht ist. Bei Aufarbeitung analog Beispiel 1 erhält man 4,4 g 4,4,5-Trimethylcyclohexan-1,3-dion. Ausbeute 28,6 % der Theorie.

## Beispiel 15

90 g einer 30 %igen NaOCH-Lösung in Methanol (entsprechend 0,5 Mol $NaOCH_3$ und 2 Mol $CH_3OH$) werden mit 200 ml Xylol versetzt und in diese Mischung innerhalb von 1 Stunde ein Gemisch aus 64 g (0,5 Mol) Octan-2-on und 43 g (0,5 Mol) Acrylsäuremethylester eingetropft. Anschließend wird das Reaktionsgemisch unter Abdestillieren von Methanol erhitzt bis die Innentemperatur im Reaktionsgefäß 130 °C beträgt. Aufarbeitung analog Beispiel 1 ergibt 80 g Rückstand entsprechend einer Rohausbeute an 4-Pentyl-cyclohexan-1,3-dion von 90 % der Theorie. Umkristallisation des Rohprodukts aus Essigsäureethylester ergibt 40 g eines Produktes vom Schmelzpunkt F = 68-70 °C, entsprechend einer Ausbeute von 45 %.

## Beispiele 16 bis 18

In den im folgenden beschriebenen 4 Versuchen wird jeweils 0,5 Mol $NaOCH_3$ in fester Form in 200 ml Xylol suspendiert und zu dieser Suspension die aus der folgenden Tabelle ersichtliche Menge Methanol zugefügt. Zu der erhaltenen Mischung wird bei 90 °C innerhalb von 30 Min. ein Gemisch aus 0,5 Mol Acrylsäureester und 0,5 Mol Diethylketon zugetropft und das Reaktionsgemisch noch 45 Min. bei 90 °C, bzw. in den Beispielen 17. und 18 unter Abdestillieren von Methanol zum Sieden erhitzt. Anschließend wird das Reaktionsgemisch mit ca. 200 ml Wasser versetzt, die wäßrige Phase auf einen pH-Wert = 3 gebracht, mit Essigsäureethylester extrahiert, getrocknet und eingeengt. Das erhaltene Rohprodukt wird zur Entfernung von nicht kristallinen Verunreinigungen mit Petrolether (Kp = 60-70 °C) und Diethylether bis zum Erhalt eines schmelzpunktkonstanten Produkts ausgerührt. Die Ausbeute an gereinigtem Produkt ist in der Tabelle angegeben.

| Beispiel | Methanolzusatz Mol(Mol% bez. Acrylsäureester) | Ausbeute |
|---|---|---|
| Vergleichs-beispiel 1 | - | 15,7 |
| 16 | 0,05 (10 %) | 78,6 |
| 17 | 0,25 (50 %) | 74,3 |
| 18 | 2,5 (500 %) | 52,8 |

Beispiel 19

6 g festes $NaOCH_3$ wird in 50 ml Toluol suspendiert, zu dieser Suspension innerhalb von 2 Stunden unter Rückflußbedingungen ein Gemisch aus 0,1 Mol Diethylketon und 0,1 Mol β-Methoxy-propionsäuremethylester zugetropft und das Reaktionsgemisch noch 6 Stunden unter Rückfluß zum Sieden erhitzt. Aufarbeitung des Reaktionsansatzes analog Beispiel 1 ergibt das 2,4-Dimethyl-cyclohexan-1,3-dion in Ausbeuten von ca. 75 % der Theorie.

Vergleichsbeispiel 2

Arbeitet man wie in Beispiel 19 beschrieben, verwendet jedoch anstelle von 0,1 Mol β-Methoxy-propionsäuremethylester 0,1 Mol Acrylsäuremethylester, so erhält man das 2,4-Dimethyl-cyclohexan-1,3-dion nur in Ausbeuten von 11,5 % der Theorie.

Vergleichsbeispiel 3

Arbeitet man wie in Beispiel 19 beschrieben, jedoch verwendet man anstelle von 50 ml Toluol 50 ml Methanol als Lösungsmittel, so erhält man das 2,4-Dimethyl-cyclohexan-1,3-dion nur in Ausbeuten von 7 % der Theorie.

Vergleichsbeispiel 4

6 g festes $NaOCH_3$ wird in 50 ml Dimethylformamid (DMF) suspendiert. Zu dieser Suspension wird innerhalb von 2 Stunden unter Rückflußbedingungen ein Gemisch aus 0,1 Mol Diethylketon und 0,1 Mol β-Methoxy-propionsäuremethylester zugetropft, das Reaktionsgemisch noch 90 Min. nachgerührt, anschließend mit 500 ml Wasser versetzt, auf einen pH-Wert von 2-3 gebracht, mit Essigsäureethylester extrahiert und eingeengt. Man erhält 10 g Rückstand, der nach NMR-Spektrum noch 2 g DMF enthält. Ausbeute ca. 57 % der Theorie.

**Ansprüche**

1. Verfahren zur Herstellung von Cyclohexan-1,3-dionen der allgemeinen Formel I

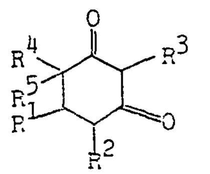

(I)

in der die Reste $R^1$, $R^3$ und $R^4$ Wasserstoff, Alkylgruppen mit 1 bis 10 C-Atomen oder eine Arylgruppe bedeuten, der Rest $R^2$ für Wasserstoff oder eine Alkylgruppe mit 1 bis 10 C-Atomen steht, der Rest $R^5$ für eine Alkylgruppe mit 1 bis 10 C-Atomen, oder eine Arylgruppe steht oder $R^4$ und $R^5$ zusammen für einen Alkylenrest mit 2 bis 10 C-Atomen stehen, oder aber $R^4$ oder $R^5$ und $R^3$ zusammen für einen Alkylenrest mit 2 bis 12 C-Atomen stehen, durch Umsetzen von Acrylsäureestern der allgemeinen Formel II

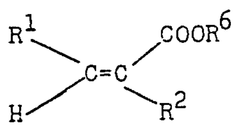

(II)

in der $R^6$ für eine Alkylgruppe mit 1 bis 4 C-Atomen steht und $R^1$ und $R^2$ die oben angegebene Bedeutung haben, mit Ketonen der allgemeinen Formel III

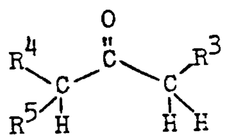

(III)

in der $R^3$ bis $R^5$ die oben angegebene Bedeutung haben, in der Flüssigphase in Gegenwart eines stark basischen Kondensationsmittels sowie in Gegenwart eines üblichen unpolaren Lösungsmittels, dadurch gekennzeichnet, daß das Reaktionsgemisch zusätzlich noch etwa 0,01-5 Mol eines Alkanols mit 1 bis 4 C-Atomen pro Mol des jeweils im Reaktionsgemisch vorhandenen Acrylsäureesters enthält und daß man das Reaktionsgemisch während und/oder nach der Zugabe der Reaktionspartner auf Temperaturen von 40 bis 200 °C erhitzt und/oder daß man den Acrylsäureester in Form seines Additionsproduktes mit einem Alkanol mit 1 bis 4 C-Atomen der allgemeinen Formel IV

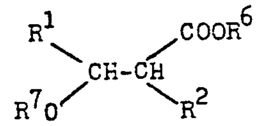

(IV)

in dem $R^7$ Alkyl mit 1 bis 4 C-Atomen bedeutet, verwendet und das Reaktionsgemisch auf Temperaturen von 40 bis 200 °C erhitzt.

2. Verfahren zur Herstellung von Cyclohexan-1,3-dionen der allgemeinen Formel I gemäß Anspruch 1, dadurch gekennzeichnet, daß man bei der Umsetzung von Acrylsäureestern der Formel II mit den Ketonen der Formel III das Reaktionsgemisch nach der Zugabe der Reaktionspartner unter Abdestillieren des im Reaktionsgemisch vorhandenen Alkanols erhitzt.

3. Verfahren zur Herstellung von Cyclohexan-1,3-dionen der allgemeinen Formel I gemäß Anspruch 1, dadurch gekennzeichnet, daß man bei der Umsetzung von Acrylsäureester-Additionsprodukten der Formel IV mit Ketonen der Formel III das Reaktionsgemisch unter Abdestillieren des im Reaktionsgemisch vorhandenen Alkanols erhitzt.

4. Bicyclo [9.3.1] pentadecan-14,15-dion.

5. 13-Methyl-bicyclo [9.3.1] pentadecan-14,15-dion.

6. 12-Methyl-bicyclo [9.3.1] pentadecan-14,15-dion.

## Claims

1. A process for the production of a cyclohexane-1,3-dione of the general formula I

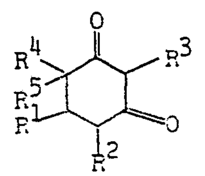

(I)

where $R^1$, $R^3$ and $R^4$ are each hydrogen, alkyl of 1 to 10 carbon atoms, or aryl, $R^2$ is hydrogen or alkyl of 1 to 10 carbon atoms, $R^5$ is alkyl of 1 to 10 carbon atoms, or aryl, or $R^4$ and $R^5$ together are alkylene of 2 to 10 carbon atoms, or $R^4$ or $R^5$ together with $R^3$ are alkylene of 2 to 12 carbon atoms, by reacting an acrylic acid ester of the general formula II

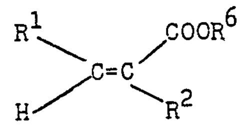

(II)

where $R^6$ is alkyl of 1 to 4 carbon atoms, and $R^1$ and $R^2$ have the above meanings, with a ketone of the general formula III

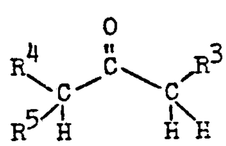

(III)

where $R^3$ to $R^5$ have the above meanings, in the liquid phase in the presence of a strongly basic condensing agent and of a conventional non-polar solvent, wherein the reaction mixture additionally contains from about 0.01 to 5 moles of an alkanol of 1 to 4 carbon atoms per mole of the particular acrylic acid ester present in the reaction mixture, and wherein the reaction mixture, during and/or after the addition of the reactants, is heated to a temperature of from 40 to 200 °C and/or the acrylic acid ester is used in the form of its adduct with an alkanol of 1 to 4 carbon atoms of the general formula IV

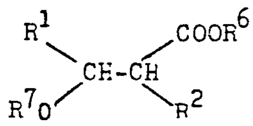

(IV)

where $R^7$ is alkyl of 1 to 4 carbon atoms, and the reaction mixture is heated to a temperature of from 40 to 200 °C.

2. A process for the production of a cyclohexane-1,3-dione of the general formula I as claimed in claim 1, wherein, in the reaction of the acrylic acid ester of the formula Ii with the ketone of the formula III, the reaction mixture, after the addition of the reactants, is heated, while distilling off the alkanol present in the reaction mixture.

3. A process for the production of a cyclohexane-1,3-dione of the general formula I as claimed in claim 1, wherein, in the reaction of the acrylic acid ester adduct of the formula IV with the ketone of the formula III, the reaction mixture is heated, while distilling off the alkanol present in the reaction mixture.

4. Bicyclo-[9,3,1]-pentadecane-14,15-dione.

5. 13-Methyl-bicyclo-[9,3,1]-pentadecane-14,15-dione.

6. 12-Methyl-bicyclo-[9,3,1]-pentadecane-14,15-dione.

**Revendications**

1. Procédé pour la préparation de cyclohexane-1,3-diones de formule générale I

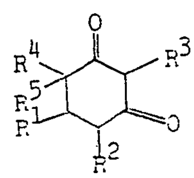

(I)

dans laquelle chacun des radicaux $R^1$, $R^3$ et $R^4$ représente un hydrogène, un groupe alcoyle à 1-10 atomes de C ou un groupe aryle, le radical $R^2$ est mis pour un hydrogène ou un groupe alcoyle à 1-10 atomes de C, le radical $R^5$ est mis pour un groupe alcoyle à 1-10 atomes de C ou un groupe aryle, ou $R^4$ et $R^5$ forment ensemble un radical alcoylène à 2-10 atomes de C ou encore $R^4$ ou $R^5$ et $R^3$ forment ensemble un radical alcoylène à 2-12 atomes de C, par réaction d'acrylates de formule générale II

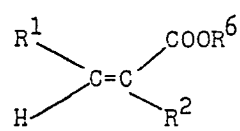

(II)

dans laquelle $R^6$ est mis pour un groupe alcoyle à 1-4 atomes de C et $R^1$ et $R^3$ ont les significations données ci-dessus, avec des cétones de formule générale III

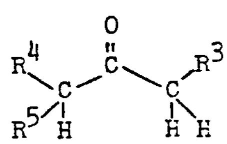

(III)

dans laquelle $R^3$ à $R^5$ ont les significations données ci-dessus, en phase liquide, en présence d'un agent de condensation fortement basique, ainsi qu'en présence d'un solvant non polaire usuel, caractérisé en ce que le mélange réactionnel contient en plus environ 0,01 à 5 mol d'un alcanol à 1-4 atomes de C par mol de l'acrylate présent dans le mélange réactionnel considéré, et en ce qu'on chauffe le mélange réactionnel, pendant et/ou après l'addition des partenaires réactionnels, à une température comprise entre 40 et 200 °C et/ou en ce qu'on utilise l'acrylate sous la forme de son produit d'addition avec un alcanol à 1-4 atomes de C de formule générale IV

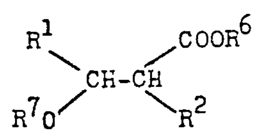

(IV)

dans laquelle $R^7$ représente un alcoyle à 1-4 atomes de C et on chauffe le mélange réactionnel à une température comprise entre 40 et 200 °C.

2. Procédé pour la préparation de cyclohexane-1,3-diones de formule générale I selon la revendication 1, caractérisé en ce qu'au cas où on fait réagir des acrylates de formule II avec des cétones de formule III, on chauffe le mélange réactionnel après l'addition des partenaires réactionnels en chassant par distillation l'alcanol présent dans la réaction.

3. Procédé pour la préparation de cyclohexane-1,3-diones de formule générale I selon la revendication 1, caractérisé en ce qu'au cas où on fait réagir des produits d'addition d'acrylate de formule IV avec des cétones de formule III, on chauffe le mélange réactionnel en chassant par distillation l'alcanol présent dans ce mélange.

4. Bicyclo [9.3.1] pentadécane-14,15-dione.

5. 13-méthyl-bicyclo [9.3.1] pentadécane-14,15-dione.

6. 12-méthyl-bicyclo [9.3.1] pentadécane-14,15-dione.